# EUROPEAN PATENT APPLICATION

(11) **EP 3 257 441 A1**
(43) Date of publication of application: **20.12.2017**
(21) Application number: 16174743.1
(22) Date of filing: 16.06.2016
(51) Int. Cl.: A61B 5/11

(54) **QUANTITATIVE SEISMOCARDIOGRAPHY**

(71) Applicant: Acarix A/S, 2800 KGS Lyngby (DK)
(72) Inventor: SCHMIDT, Samuel Emil, DK-9210 AALBORG SØ (DK); SØGAARD, Peter, 2300 KØBENHAVN (DK); STRUIJK, Johannes Jan, 9575 TERNDRUP (DK)
(74) Representative: Brann AB

(57) **Abstract**

A quantifying of the function of a beating heart is disclosed, in which a signal is recorded with an accelerometer placed on the chest of a person. A plurality of segments of the signal are formed, which are aligned and filtered with a band-pass filter having a lower cutoff frequency below 1 Hz and an upper cut-off frequency in the range 100-500 Hz. A mean segment is then determined, in which a first temporal feature is determined. A measure is then determined based on at least one of the signal strength of the first temporal feature and the location in time of the first temporal feature. The determined measure is then provided as output information.

## Description

### Technical field of the Invention

The present invention generally relates to techniques for diagnostic purposes relating to cardiovascular function or fitness, and in particular to techniques for assisting in diagnosing systolic and diastolic dysfunction that could lead to heart failure.

### Background of the Invention

Diastolic dysfunction is a frequent occurring heart defect in persons older than 45 years. Diastolic dysfunction can lead to severe heart failure and is associated with increased mortality. Current methods for diagnosis of diastolic dysfunction are complicated and expensive, which typically means that they are used only on patients with high risk of diastolic dysfunction.

Seismocardiography (SCG) is the analysis of sub-audible low-frequency vibrations at the chest wall caused by the beating heart. More generally, SCG relates to non-invasive measurement of accelerations in the chest wall produced by myocardial movement. Heart sounds are audible components of the chest wall vibrations that typically are above 40-60 Hz, while SCG vibrations typically are below 5 Hz.

SCG is typically measured using an accelerometer. However, when an accelerometer is used, both low frequency SCG components and audible components are simultaneously sampled. The SCG components and the audible components reveal different cardiovascular functions, thus enabling different approaches to diagnosing a cardiovascular function. For example, SCG is typically suitable for estimation of time intervals between features in the cardiac cycle, while heart sounds are appropriate for detection of murmurs caused by flow disturbances.

When using an accelerometer, the heart sounds or audio components in the accelerometer signal are dominated by the high intensity of the low-frequency vibrations, or SCG waves. If the accelerometer signal is high pass filtered, for example with a lower cutoff of 50 Hz, the heart sounds are revealed. In the heart sound, the most dominating sounds are the first heart sound (S1) and second heart sounds (S2), which are related to the mitral valve closure (MC) and the aortic valve closure (AC), respectively.

A problem for General Practitioners is that heart failure shares symptoms with other common diseases, such as respiratory disease. Thus, there is a need for a reliable or accurate tool that can assist in diagnosing or determining a probability of heart failure.

### Object of the Invention

An object of the present invention is to provide an improved tool for quantifying the function of a beating heart. It is also an object to provide an improved technology for identifying heart failure.

### Summary of the Invention

According to a first aspect, the aforementioned objects are accomplished by a method for quantifying the function, cardiovascular function, or cardiovascular fitness of a beating heart. The method comprises: obtaining a plurality of segments of a signal recorded with an accelerometer placed on the chest of a person for measuring accelerations and vibrations of the chest wall of the person caused by myocardial movement, wherein each segment covers, or corresponds to, a cardiac cycle. The method further comprises: aligning the plurality of segments, filtering the plurality of segments with a band-pass filter having a lower cutoff frequency below 5 Hz, preferably below 1 Hz, and an upper cut-off frequency in the range 100-500 Hz, and determining a mean segment based on the plurality of segments. The method further comprises: determining a first temporal feature in the filtered mean segment, determining a measure based on at least one of the signal strength of the first temporal feature and the location in time of the first temporal feature, and providing output information based on the determined measure.

Here, quantifying the function is understood to encompass quantifying heart failure, or determining an indication of heart failure. It is also understood to encompass determining a myocardial performance index or other contraction related measures, such as the amplitude of the S1 heart sound. The specified band-pass filtering has the effect that the temporal feature is determined from mean segment including both low-frequency SCG components and audible components, as compared to when only low-frequency SCG components are considered. It has been found the specified band-pass filtering contributes to a reliable, and thus improved, quantifying of heart failure.

Throughout these specifications, a temporal feature may correspond to a feature or stage in a cardiac cycle. A temporal feature may correspond to a peak, valley, local extremum, local minima, local maxima, maximal change, maximal increase, or maximal decrease of the filtered mean segment. A measure may, throughout these specifications, correspond to a signal strength or an amplitude, or a difference in time. The signal strength of a temporal feature may correspond to an acceleration.

According to a second aspect, the objects are achieved by system for quantifying the function, cardiovascular function, or cardiovascular fitness, of a beating heart. The system comprises:(A) an accelerometer configured to be placed on the chest of a person for measuring accelerations and vibrations of the chest wall of the person caused by myocardial movement, and (B) a processor operatively connected to the accelerometer. The processor is configured to: obtain a plurality of segments of a signal recorded with the accelerometer, wherein each segment covers, or corresponds to, a cardiac cycle. The processor is also configured to: align the plurality of segments, filter the plurality of segments with a band-pass filter having a lower cutoff frequency below 1 Hz and an upper cut-off frequency in the range 100-500 Hz, and determine a mean segment based on the plurality of segments. The processor is further configured to: determine a first temporal feature in the mean segment, determine a measure based on at least one of the signal strength of the first temporal feature and the location in time of the first temporal feature, and provide output information based on the determined measure.

In the above aspects, to obtain a plurality of segments of a signal may comprise: obtaining the signal and forming the plurality of segments from the signal.

According to a third aspect, the objects are achieved by a system for quantifying the function, cardiovascular function, or cardiovascular fitness of a beating heart. The system comprises: an accelerometer configured to be placed on the chest of a person for obtaining a signal representing accelerations and vibrations of the chest wall of the person caused by myocardial movement, and a segmentation module for forming a plurality of segments from the signal, wherein each segment covers, or corresponds to, a cardiac cycle. It further comprises: an align module for aligning the plurality of segments, a filter module for filtering the plurality of segments with a band-pass filter having a lower cutoff frequency below 1 Hz and an upper cut-off frequency in the range 100-500 Hz, and a first calculation module for determining a mean segment based on the plurality of segments. The system also comprises: a second calculation module for determining a first temporal feature in the mean segment and, a third calculation unit for determining a measure based on at least one of the signal strength of the first temporal feature and the location in time of the first temporal feature, and an output module for providing output information based on the determined measure.

According to a fourth aspect, the objects are achieved by a computer program product for being used in a system comprising: (A) an accelerometer for being placed on, or configured to be placed on, the chest of a person for measuring accelerations and vibrations of the chest wall of the person caused by myocardial movement, and (B) a processor operatively connected with the accelerometer. The computer program product comprising program code instructions configured to, when executed by the processor of the system, cause the processor to: obtain a signal with the accelerometer, and forming a plurality of segments from the signal, wherein each segment covers, or corresponds to, a cardiac cycle. The program code instructions further causes the processor to: align the plurality of segments, filter the plurality of segments with a band-pass filter having a lower cutoff frequency below 1 Hz and an upper cut-off frequency in the range 100-500 Hz, and determine a mean segment based on the plurality of segments. The program code instructions are further configured to cause the processor to: determine a first temporal feature in the mean segment, determine a measure based on at least one of the signal strength of the first temporal feature and the location in time of the first temporal feature, and provide output information based on the determined measure.

According to a fifth aspect, the objects are achieved by a non-transient memory on which a computer program product according to the fourth aspect is stored.

In the different aspects above, the output information may represent the actual determined measure. Alternatively, the output information may represent a score based on the determined measure. Additionally or alternatively, the aligning may be performed prior to the filtering, and the filtering may be performed prior to determining the mean segment.

In the method of the first aspect, the accelerometer may be placed on the chest of a person and attached to the skin of the person by an adhesive for measuring the accelerations and vibrations. The systems of the second, third and fourth aspects may further comprise an adhesive patch configured for supporting the accelerometer and for being attached to the skin of the person. By attaching the accelerometer to the skin, the quality of the recorded signals is improved.

### Detailed description

The different aspects described above may be modified as described below.

The step of obtaining a segment of a signal recorded with an accelerometer may comprise: recording a signal with an accelerometer placed on the chest of a person for measuring accelerations and vibrations of the chest wall of the person caused by myocardial movement, wherein the signal is recorded over a period of time covering a plurality of cardiac cycles of the person. The step further may comprise: dividing the recorded signal into the plurality of segments, wherein each segment covers a single cardiac cycle.

The aligning the plurality of segments may comprise: determining a heart sound in each of the plurality of segments, and aligning the plurality of segments by the determined heart sound of each segment. The heart sound may be the first heart sound (S1) or the second heart sound (S2). The first heart sound (S1) may correspond to the closing of the atrioventricular valves. The second heart sound (S2) may correspond to the closing of the semilunar valves. It has been found that the specified aligning, together with the specific filtering, contributes to a reliable quantifying of heart failure.

The measure may correspond to, or be based on, the signal strength of or at the first temporal feature. For example, the measure may correspond to the amplitude of the first heart sound (S1). This has been found to be an advantageous measure to study when examining cardiovascular fitness.

Further, determining a measure may comprise: determining the signal strength of the first temporal feature. The first temporal feature may correspond to: the aortic valve opening (AO) of a heart cycle, the atrial systole (AS) of a heart cycle, the isometric contraction (IM) of a heart cycle, or the rapid ventricular ejection or rapid emptying event (RE) of a heart cycle. It has been found that these features are particularly suitable for quantifying heart failure. A possible explanation is that heart failure cause weaker cardiac contraction, and thus influences the signal strength.

The method according to the first aspect may further comprise: determining a second temporal feature in the filtered mean segment, and wherein determining a measure is further based on at least one of the signal strength of the second temporal feature and on the location in time of the second temporal feature. By having two or more temporal features, additional measures can be used, thus contributing to an improved technology for identifying heart failure.

The measure may further be based on the location in time of the first temporal feature and on the location in time of the second temporal feature. Determining a measure may comprise: determining the difference between the location in time of the first temporal feature and the location in time of the second temporal feature, wherein the measure is based on the determined difference. Alternatively, determining a measure may comprise: determining the time interval between the first temporal feature and the second temporal feature, wherein the measure is based on the determined time interval.

Alternatively or additionally, the measure may be based on the signal strength of the first temporal feature and on the signal strength of the second temporal feature. The signal strength of the first temporal feature may be normalized by the signal strength of the second temporal feature. Determining a measure may comprise: determining the difference or ratio between the signal strength of the first temporal feature and the signal strength of the second temporal feature, wherein the measure is based on the determined difference or ratio.

The first temporal feature may correspond to the mitral valve closure (MC) and the second temporal feature may correspond to the rapid ventricular ejection or rapid emptying event (RE). Alternatively, the first temporal feature may correspond to the atrial systole (AS) in the cardiac cycle and the second temporal feature may correspond to the mitral valve closure (MC) in the cardiac cycle. Alternatively, the first temporal feature may correspond to the aortic valve closing (AC) and the second temporal feature may correspond to the mitral valve opening (MO). Alternatively, the first temporal feature may correspond to the aortic valve opening (AO) and the second temporal feature may correspond to the aortic valve closing (AC).Alternatively, the first temporal feature may correspond to the mitral valve closure (MC) and the second temporal feature may correspond to the aortic valve opening (AO). It has been found that these features are particularly suitable for quantifying heart failure. A possible explanation is that heart failure cause slower cardiac contraction, and thus influences the timing in the heart cycle.

The method, or determining the first temporal feature, may further comprise: determining a first point in time in the mean segment corresponding to the onset of a heart sound. Determining the first temporal feature may further comprise: determining the first temporal feature relative to the first point in time. Similarly, determining the second temporal feature may further comprise: determining the second temporal feature relative to the first point in time. The heart sound may be the first heart sound (S1) or the second heart sound (S2). As mentioned above, the first heart sound (S1) may correspond to the closing of the atrioventricular valves and the second heart sound (S2) may correspond to the closing of the semilunar valves.

If the heart sound is the first heart sound (S1), determining a first temporal feature may comprise: determining the first local minima (IM) subsequent to the first point in time, and assigning the first local minima to represent the isovolumic movement (IM), and/or determining the maximum negative deviation (MC) prior to the first local minima (IM) subsequent to the first point in time, and assigning the maximum negative deviation to represent the mitral valve closure (MC). Alternatively or additionally, determining a first temporal feature may comprise: determining the global maxima subsequent to the first point in time, and assigning the global maxima to represent the aortic valve opening (AO), and/or determining the global maxima (RE) subsequent to the global minima (IC) subsequent to the global maxima (AO) subsequent to the first point in time, and assigning the global maxima (RE) subsequent to the global minima to represent the rapid ventricular ejection or rapid emptying event (RE). Here, a global extremum on one side of an event only relate to the local extrema on the same side of the event. It has been found that these definitions are advantageous for determining an indication of heart failure.

The lower cutoff frequency of the band-pass filter may be below 0.5 Hz, 0.2 Hz, or approximately 0.1 Hz. The upper cutoff frequency may be the range of 150-250 Hz, 175-225 Hz, or approximately 200 Hz, or in one of the ranges 100-150 Hz, 150-200 Hz, 200-250 Hz, and 250-300 Hz. These frequencies for providing the SCG signal have been found to give reliable results.

The method may further comprise: determining a heart rate of the beating heart. Similarly, the processor may be configured to: determine a heart rate of the beating heart, and the computer program product may comprising program code instructions configured to, when executed by the processor of the system, cause the processor to: determine a heart rate of the beating heart. The heart rate may be determined based on the signal recorded with the accelerometer. The heart rate may indicate the number of contraction of the heart per minute or another suitable period of time.

Determining the measure may further be based on the heart rate. For example, determining the measure may comprise: determining the difference between the location in time of a first temporal feature and the location in time of a second temporal feature and dividing the difference with the heart rate. It is contemplated that by taking the heart rate into account, the measure can be determined more accurately for persons having a high heart rate at rest and for persons that are active or exercising when the signal is recorded with the accelerometer.

The system of the above aspects may comprise a non-transient memory storing program code instructions that, when executed by the processor, configures the processor to perform the described steps and/or have the described functions.

The system may comprise a smart-phone. The processor and/or the non-transient memory may be integral parts of the smart-phone. Further, the accelerometer may be an integral part of the smart-phone. The system may also comprise a casing or holder for supporting the smart-phone, and the casing or holder may comprise an adhesive patch configured for attaching the casing or holder to the skin of the person. Alternatively to the accelerometer being an integral part of the smart-phone, the accelerometer may form part of an auxiliary unit configured to communicate with the smart-phone by wire or wirelessly, such as a fitness band that can be strapped around the chest of person.

Providing the output information may further comprise: storing the plurality of segments, the mean segment and/or the measure in the non-transient memory or in the auxiliary non-transient memory. The auxiliary non-transient memory may form part of computer server system, which may be at a remote location.

Providing the output information may further comprise: providing a previously obtained measure and the output information may further be based on the previously obtained measure. The previously obtained measure may be stored in the non-transient memory or in the auxiliary non-transient memory. The output information may be based on the difference between the measure and the previously obtained measure. For example, the output information may be the difference in amplitude between a first heart sound (S1) and a previously obtained heart sound.

Additionally or alternatively, providing the output information may further comprise: providing a previously obtained mean segment and the output information may further be based on the mean segment and the previously obtained mean segment. The previously obtained mean segment may be stored in the non-transient memory or in the auxiliary non-transient memory. The output information may comprise: a graph overlying the mean segment with the previously obtained mean segment. More specifically, the output information may comprise: a graph overlying a portion of the mean segment and the corresponding portion of the previously obtained mean segment. For example, the portion may cover the first heart sound (S1). The graph may be displayed on the screen of abovementioned smart-phone.

The previously obtained measure or previously obtained mean segment may have been determined in the same manner as the measure or the mean segment, or by the same steps as performed for determining the measure or the mean segment. The previously obtained measure or previously obtained mean segment may have been determined at an earlier point in time, such as more than five days or ten days prior to determining the measure or the mean segment. This is particularly advantageous when studying how cardiovascular fitness changes during an extended period of training or exercising.

Further advantages with and features of the different aspects will be apparent from the following description of the drawing.

### Brief description of the drawings

A more complete understanding of the abovementioned and other features and advantages of the present invention will be apparent from the following detailed description of the drawings, wherein:
Fig.1 is a schematic illustration of an embodiment of a system 12 for quantifying the function of a beating heart,
Fig.2 is a flow chart illustrating the basic steps of a method employed in the system described in relation to Fig.1,
Fig.3 is a flow chart illustrating sub-steps and additional steps of a method based on the method described in relation to Fig.2, and
Figs.4a and 4b shows a segment that has been filtered by a band-pass filter and to a high-pass filter, respectively.

### Detailed description of drawings

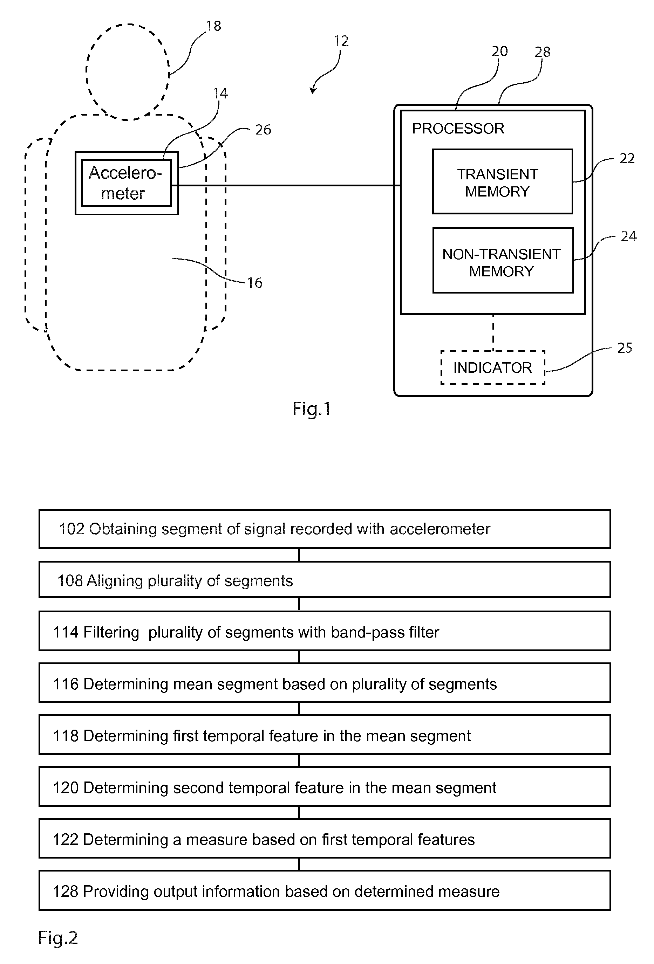

Fig.1 schematically illustrates an embodiment of a system 12 for quantifying the function of a beating heart, or more specifically for determining an indication of heart failure. The system 12 has an accelerometer 14 that can be placed on the chest of a person 18 and for measuring vibrations of the chest wall caused by movements of the heart. A processor 20 is connected with the accelerometer 14. The processor 20 has a transient memory 22 which can store a signal received from the accelerometer 14, and by which it can execute program code instructions. The system 12 comprises a support 26 that supports the accelerometer 14 and a housing 28 that accommodates the processor 20. The system 12 also has a non-transient memory 24 storing program code instructions for the processor 20. For example, the system 12 as a whole can be an integral part of a smart-phone, or all parts except the accelerometer 20 and the support 26 can form part of a smart-phone. In one embodiment, the accelerometer is an integrated accelerometer of a smart-phone.

In one embodiment of the system 12, it additionally has an indicator 25 operatively connected with the processor 20. The indicator 25 can, for example, have an LCD display, or the like, that can display output information from the processor 20, such as a number.

The program code instructions in the non-transient memory cause the processor 20 to perform a method that is shown in Fig.2. The accelerometer is placed on the chest of a person and a signal is recorded. A plurality of segments is then obtained 102, where each segments corresponds to a heartbeat or a cardiac cycle. This is followed by an alignment 108 and a filtering 114. Here, a band-pass filter is employed having a lower cut-off frequency of approximately 0.1 Hz, and an upper cut-off frequency of approximately 200 Hz. Subsequently, a mean segment is determined 118 from the plurality of segments.

With the mean segment formed, a temporal feature is determined 120. The temporal feature in turn is used to determine 122 a measure. Examples of temporal features and measures are described below. Output information is then provided 128 based on the determined measure. In one embodiment, the output information is a number that is displayed on the abovementioned indicator 25.

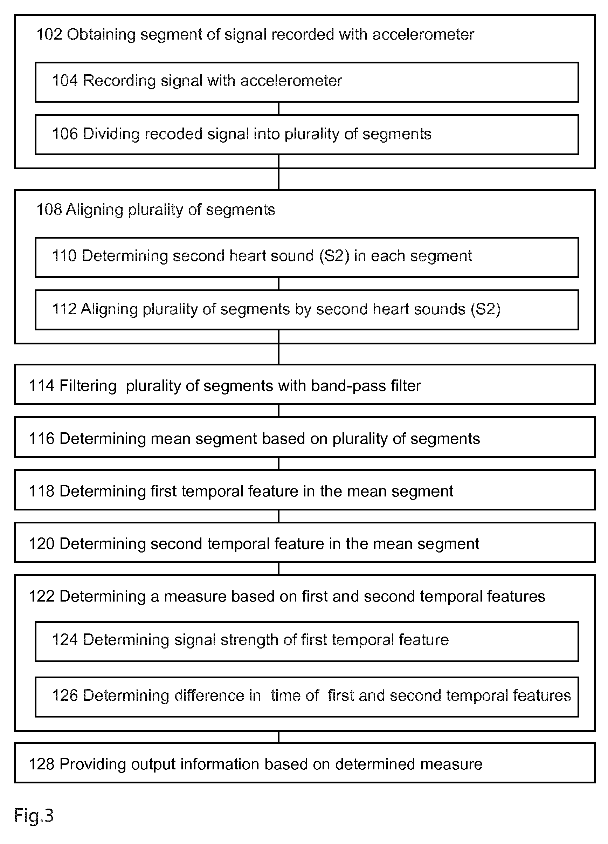

Further details of the method are shown in the flow chart of Fig.3. The step of obtaining 102 the plurality of segments includes the sub-steps of recording 104 the signal with the accelerometer, and dividing 106 the recorded signal into the plurality of segments, for example by a technique as described in US 8235912 B2 and US 8469896 B2 relying on audible components of the accelerometer signal.

In an alternative embodiment, an electrocardiography (ECG) signal is acquired simultaneously to the accelerometer signal, and the ECG signal is used for the segmentation of the latter. For example, a segmentation as described in Jensen et al. (Computing in Cardiology 2014; 41:29-32) can be used.

When obtaining 102 the plurality of segments, a method similar to the method described in Jensen et al. is employed to remove noisy segments. A high-pass filter with a lower cut-off of 65 Hz is applied to the segments and the onset of the first heart sound S1 is then determined by a known technique. Similarly, a high-pass filter with a lower cut-off of 50 Hz is applied to the segments and the onset of the second heart sound S2 is then determined by a known technique. The segments are then aligned according to the determined second heart sound S2. In alternative embodiment, the first heart sound is used instead.

The mean segment is determined 116 by summing the aligned segments to a single segment and dividing the resulting signal strength by the number of segments in the sum.

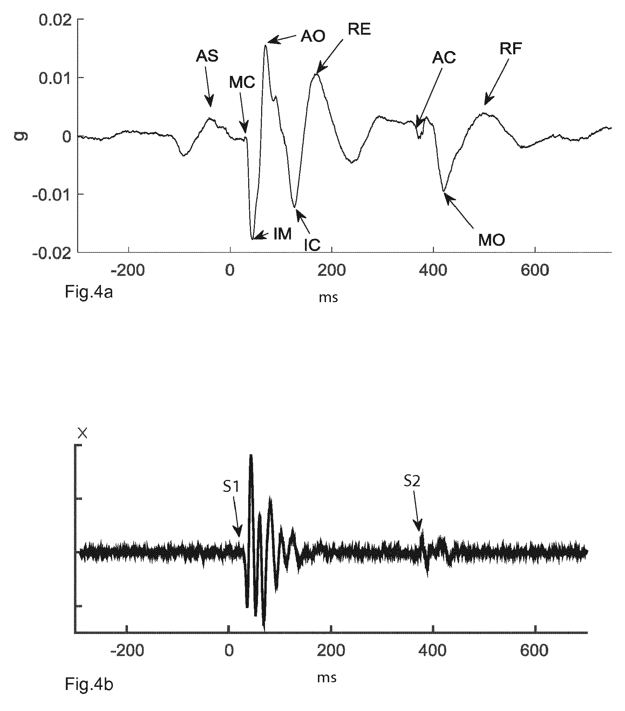

Fig.4a shows a segment that has been subjected to the above described band-pass filtering. The abscissa represents an acceleration in g (ms⁻²) and the ordinate the time in milliseconds (ms). The zero point of the ordinate corresponds to the R peak in a simultaneously recorded segment of an ECG signal. A number of temporal features are indicated in Fig.4a, which are further described below.

Fig.4b shows a segment that has been subjected to a high-pass filter with a lower cut-off of 50 Hz, as described above. The abscissa represents the signal strength X (no unit) and the ordinate the time in milliseconds (ms). The latter has been aligned by the simultaneously recorded segment of an ECG signal in the same manner as described in relation to Fig.4a. The onset of the first heart sound (S1) and the second heart sound (S2) are indicated in Fig.4b.

In the steps of determining 118 the first temporal feature and determining 120 the second temporal feature 120 in the mean segment, the following temporal features are identified in the mean segment: the mitral valve closure (MC), isovolumic movement (MO), aortic valve opening (AO), the rapid ventricular ejection (RE), the aortic valve closure (AC), the mitral valve opening (MO), the rapid ventricular filling (RF), and the atrial systole (AS).

For example, with the first point in time determined to be the onset of the first heart sound (S1), see Fig.4b, the isovolumic movement (IM) is determined as the first local minima (IM) subsequent to the first point in time, the mitral valve closure (MC) is determined as the maximum negative deviation (MC) prior to the first local minima (IM) subsequent to the first point in time, the aortic valve opening (AO) is determined as the global maxima subsequent to the first point in time, and the rapid ventricular ejection (RE) is determined as the global maxima (RE) subsequent to the global minima (IC) subsequent to the global maxima (AO) subsequent to the first point in time. For example, the first point in time can be determined by a technology similar to the technology described in US 8235912 B2 and US 8469896 B2.

One measure that is determined is the amplitude or signal strength of the above mentioned temporal features. For example, the signal strengths of the following temporal features can be determined, the aortic valve opening (AO), the atrial systole (AS), the isometric contraction (IM), and the rapid ventricular ejection (RE) of a heart cycle.

Another measure that is determined is based on the locations in time, or positions on the abscissa of the Fig.4a, of a first temporal feature and a second temporal feature. The measure is then determined as the difference in time between a first temporal feature and a second temporal feature, wherein the measure is based on the determined difference. The following measures can be determined, the difference in time between the mitral valve closure (MC) and the rapid ventricular ejection (RE), the difference in time between the atrial systole (AS) and the mitral valve closure (MC), the difference in time between the aortic valve closing (AC) and the mitral valve opening (MO), the difference in time between the aortic valve opening (AO) and the aortic valve closing (AC), and the difference in time between the mitral valve closure (MC) and the aortic valve opening (AO).

Output is then provided 128 in the form of values that are displayed on an LCD display of the indicator 206, where the values represents the determined signal strengths and differences in time in the examples above.

### Proof of concept

A custom lightweight 8 g piezoelectric accelerometer was developed for acquisition of the SCG signals. The low weight provides a better signal and the miniaturization allows for the accelerometer to be incorporated in another device. The accelerometer was used in a system as described above in relation to Figs.1-4.

It should be noted that the system identifies AO and MO and the resulting measures are not consistent. However, despite this disadvantage, AO and MO were used, since they have diagnostic importance.

The system was used on a first group of healthy subjects and on a second group of heart failure (HF) subjects undergoing pacemaker optimization. The majority of the heart failure subjects suffered from diastolic dysfunction. As is evident from Table 1 below, the amplitudes, or signal strength, and differences in time, or time intervals, of the temporal features provide a clear discrimination between heart failure patients and the normal subjects included.

**Table 1 shows average values and classification performance of the measures using the area under the receiving operating curve (AUC). The measures are sorted in decreasing classification performance.**

| Measure | Normal subjects Mean (STD) | HF subjects Mean (STD) | Classification AUC |
|---|---|---|---|
| Amplitudes | [mg] | [mg] | |
| AO | 21.9 (13.1) | 3.0 (2.2) | 98.0% |
| AS | 5.4 (2.5) | 1.5 (0.9) | 97.1% |
| IM | -23.7 (14.1) | -3.5 (3.5) | 95.0% |
| RE | 7.3 (4.1) | 2.6 (2.5) | 86.1% |
| Time intervals | [ms] | [ms] | |
| MC-RE | 140.6 (26.2) | 184.8 (27.8) | 89.5% |
| AS-MC | 65.6 (39.6) | 137.9 (71.1) | 83.4% |
| AC-MO | 43.1 (13.3) | 59.7 (24.8) | 74.6% |
| AO-AC | 296.6 (38.5) | 311.7 (57.2) | 63.2% |
| MC-AO | 41.7 (19.7) | 43.8 (31.8) | 42.2% |

### Feasible modifications of the Invention

The invention is not limited only to the embodiments described above in relation to the drawings, which primarily have an illustrative and exemplifying purpose. This patent application is intended to cover all adjustments and variants of the preferred embodiments described herein, thus the present invention is defined by the wording of the appended claims and the equivalents thereof. Thus, the equipment may be modified in all kinds of ways within the scope of the appended claims and the detailed description.

## Claims

1. A method for quantifying the function of a beating heart, wherein the method comprises:
- obtaining a plurality of segments of a signal recorded with an accelerometer placed on the chest of a person for measuring accelerations and vibrations of the chest wall of the person caused by myocardial movement, wherein each segment covers a cardiac cycle,
- aligning the plurality of segments, filtering the plurality of segments with a band-pass filter having a lower cutoff frequency below 1 Hz and an upper cut-off frequency in the range 100-500 Hz, and determining a mean segment based on the plurality of segments,
- determining a first temporal feature in the mean segment,
- determining a measure based on at least one of the signal strength of the first temporal feature and the location in time of the first temporal feature, and
- providing output information based on the determined measure.

2. The method according to claim 1, wherein obtaining a segment of a signal recorded with an accelerometer comprises:
- recording a signal with an accelerometer placed on the chest of a person for measuring accelerations and vibrations of the chest wall of the person caused by myocardial movement, wherein the signal is recorded over a period of time covering a plurality of cardiac cycles of the person, and
- dividing the recorded signal into the plurality of segments, wherein each segment covers a single cardiac cycle.

3. The method according to claim 1 or 2, wherein aligning the plurality of segments comprises: determining the second heart sound (S2) in each of the plurality of segments, and aligning the plurality of segments by the determined second heart sound (S2) of each segment.

4. The method according to any of the claims 1-3, wherein the measure is based on the signal strength of the first temporal feature.

5. The method according to claim 4, wherein the first temporal feature corresponds to:
- the aortic valve opening (AO) of a heart cycle,
- the atrial systole(AS) of a heart cycle,
- the isometric contraction (IM) of a heart cycle, or
- the rapid ventricular ejection or rapid emptying event (RE) of a heart cycle.

6. The method according to any of the claims 1-5, wherein the method further comprises:
- determining a second temporal feature in the mean segment, and wherein
determining a measure is further based on at least one of the signal strength of the second temporal feature and on the location in time of the second temporal feature.

7. The method according to claim 6, wherein the measure is based on the location in time of the first temporal feature and on the location in time of the second temporal feature, and wherein determining a measure comprises: determining the difference between the location in time of the first temporal feature and the location in time of the second temporal feature, and wherein the measure is based on the determined difference.

8. The method according to any of the claims 6-7, wherein:
- the first temporal feature corresponds to the mitral valve closure (MC) and the second temporal feature corresponds to the rapid ventricular ejection or rapid emptying event (RE), or
- the first temporal feature corresponds to the atrial systole(AS) in the cardiac cycle and the second temporal feature corresponds to the mitral valve closure (MC) in the cardiac cycle, or
- the first temporal feature corresponds to the aortic valve closing (AC) and the second temporal feature corresponds to the mitral valve opening (MO), or
- the first temporal feature corresponds to the aortic valve opening (AO) and the second temporal feature corresponds to the aortic valve closing (AC), or
- the first temporal feature corresponds to the mitral valve closure (MC) and the second temporal feature corresponds to the aortic valve opening (AO).

9. The method according to claim 1 or 8, wherein the method further comprises: determining a first point in time in the mean segment corresponding to the onset of a heart sound (Sl or S2), and determining a first temporal feature and/or the second temporal feature further comprises: determining the first temporal feature and/or the second temporal feature relative to the first point in time.

10. The method according to any of the claims 1-9, wherein the lower cutoff frequency is below 0.5 Hz, 0.2 Hz, or approximately 0.1 Hz.

11. The method according to any of the claims 1-10, wherein the upper cutoff frequency is in the range of 150-250 Hz, 175-225 Hz, or approximately 200 Hz, or in one of the ranges 100-150 Hz, 150-200 Hz, 200-250 Hz, and 250-300 Hz.

12. A system for quantifying the function of a beating heart, wherein the system comprises:
(A) an accelerometer configured to be placed on the chest of a person for measuring accelerations and vibrations of the chest wall of the person caused by myocardial movement,
(B) a processor operatively connected to the accelerometer and configured to:
- obtain a plurality of segments of a signal recorded with the accelerometer,
- align the plurality of segments, filter the plurality of segments with a band-pass filter having a lower cutoff frequency below 1 Hz and an upper cut-off frequency in the range 100-500 Hz, and determine a mean segment based on the plurality of segments,
- determine a first temporal feature in the mean segment,
- determine a measure based on at least one of the signal strength of the first temporal feature and the location in time of the first temporal feature, and
- provide output information based on the determined measure.

13. A computer program product for being used in a system comprising: (A) an accelerometer for being placed on the chest of a person for measuring accelerations and vibrations of the chest wall of the person caused by myocardial movement, and (B) a processor operatively connected with the accelerometer, the computer program product comprising program code instructions configured to, when executed by the processor of the system, cause the processor to:
- obtain a signal with the accelerometer and form a plurality of segments from the signal, wherein each segment covers, or corresponds to, a cardiac cycle.
- record a plurality of segments of a signal with the accelerometer,
- align the plurality of segments, filter the plurality of segments with a band-pass filter having a lower cutoff frequency below 1 Hz and an upper cut-off frequency in the range 100-500 Hz, and determine a mean segment based on the plurality of segments,
- determine a first temporal feature in the mean segment,
- determine a measure based on at least one of the signal strength of the first temporal feature and the location in time of the first temporal feature, and
- provide output information based on the determined measure.

14. A non-transient memory on which a computer program product according to claim 13 is stored.
